# EUROPEAN PATENT SPECIFICATION

(11) **EP 1 493 441 B1**
(45) Date of publication and mention of the grant of the patent: **31.08.2011**
(21) Application number: 03745958.3
(22) Date of filing: 08.04.2003
(51) Int. Cl.: A61K 31/426, A61P 1/00, C07D 277/56, A61K 31/34, A61K 31/38, A61K 31/42

(54) **THERAPEUTIC AGENT FOR FOOD COMPETENCE DISORDER IN STOMACH**
THERAPEUTISCHES MITTEL FÜR NAHRUNGSKOMPETENZSTÖRUNGEN IM MAGEN
AGENT THERAPEUTIQUE TRAITANT LES TROUBLES STOMACAUX DE COMPETENCE ALIMENTAIRE

(30) Priority: 08.04.2002 JP 2002104894
(43) Date of publication of application: 05.01.2005
(60) Divisional application: 10010621.0
(73) Proprietor: ZERIA PHARMACEUTICAL CO., LTD., Tokyo 103-8351 (JP)
(72) Inventor: MATSUNAGA, Yugo, Zeria Pharmaceutical Co., Ltd., Osato-gun, Saitama 360-0111 (JP); UEKI, Shigeru, Zeria Pharmaceutical Co., Ltd., Osato-gun, Saitama 360-0111 (JP); HIGASHINO, Raita, Zeria Pharmaceutical Co., Ltd., Osato-gun, Saitama 360-0111 (JP); KAWACHI, Masanao, Zeria Pharmaceutical Co., Ltd., Osato-gun, Saitama 360-0111 (JP); KATO, Hiroki, Zeria Pharmaceutical Co., Ltd., Tokyo 103-8351 (JP); KOBAYASHI, Shiro, Zeria Pharmaceutical Co., Ltd., Tokyo 103-8351 (JP)
(74) Representative: Hartz, Nikolai
(86) International application number: PCT/JP2003/004445
(87) International publication number: WO 2003/084537

(56) References cited:
- EP-A1- 0 870 765
- EP-A1- 0 994 108
- WO-A-00/30640
- WO-A1-01/56573
- WO-A1-02/20010
- JP-A- 10 212 271
- DATABASE BIOSIS [Online] BIOSCIENCES INFORMATION SERVICE, PHILADELPHIA, PA, US; October 1998 (1998-10), ITO YUSHI: "Modulatory mechanisms involved in the parasympathetic excitatory neuro-effector transmission in the airway and gastrointestinal tract: Perspective for a new drug creation" XP002447256 Database accession no. PREV199800515863 & FOLIA PHARMACOLOGICA JAPONICA, vol. 112, no. SUPPL. 1, October 1998 (1998-10), pages 28P-31P, ISSN: 0015-5691
- KIBBLE A: "DIGESTIVE DISEASE WEEK" IDRUGS, CURRENT DRUGS LTD, GB, vol. 2, no. 7, 1999, pages 639-642, XP000995743 ISSN: 1369-7056
- MORITA H.: 'Possible involvement of M5 muscarinic receptor in the enhancing actions of the novel gastroprokinetic agent Z-338 on nifedipine-sensitive voltage-dependent Ca2+ currents in guinea pig stomach' JAPANESE JOURNAL OF PHARMACOLOGY vol. 89, no. 4, 2002, pages 356 - 365, XP002962154
- KANEMOTO Y.: 'An electrophysiological study of muscarinic and nicotinic receptors of rat paratracheal ganglion neurons and their inhibition by Z-338' BRITISH JOURNAL OF PHARMACOLOGY vol. 135, no. 6, 2002, pages 1403 - 1414, XP002962155
- OGISHIMA M.: 'Z-338 facilitates acetylcholine release from enteric neurons due to blockade of muscarinic autoreceptors in guinea pig stomach' JOURNAL OF PHARMACOLOGY AND EXPERIMENTAL THERAPEUTICS vol. 294, no. 1, 2000, pages 33 - 37, XP002962156
- NAKAJIMA T.: 'Z-338, a newly synthesized carboxyamide derivative, stimulates gastric motility through enhancing the excitatory neurotransmission' JOURNAL OF SMOOTH MUSCLE RESEARCH vol. 36, no. 2, 2000, pages 69 - 81, XP002962157
- FURUTA S.: 'LC determination of Z-338, novel gastroprokinetic agent in dog plasma by SCX solid phase extraction' JOURNAL OF PHARMACEUTICAL AND BIOMEDICAL ANALYSIS vol. 25, no. 3-4, 2001, pages 599 - 603, XP002962158

## Description

### Technical Field

The present invention relates to a therapeutic agent for alleviating symptoms caused by impaired gastric accommodation.

### Background of the Invention

Therapeutic agents for motility disorder of gastrointestinal tract that have been clinically used include, for example, dopamine antagonists such as domperidone and metoclopramide; opiate agonist such as trimebutine maleate; 5HT₃ antagonist/5HT₄ agonist such as cisapride; and acetylcholine agonist such as acetylcholine chloride. The present inventors have also found that a specific aminothiazole derivatives and benzoylamine derivatives have excellent activity for improving gastrointestinal tract motility, and thus previously applied for a patent (WO96/36619 and JP-A-10-212271).

These traditional therapeutic agents for motility disorder of gastrointestinal tract have been screened on the basis of improving potential for stomach motility, more specifically on the basis of contracting activity at gastric antrum by animal experiments, and clinical efficacy thereof has been confirmed by improving effect on delayed gastric emptying.

However, in recent years, it has been clarified that feeling of early satiety and bloating after ingestion of meal can not be improved sufficiently by enhancing gastric emptying. It has been found that relaxation of gastric fundus, such as improvement of impaired gastric accommodation is necessary to improve these symptoms but not by improvement of stomach motility; that is, gastric emptying (Aliment Pharmacol. Ther. 1998: 12: 761-766).

As to Cisapride, one of traditional therapeutic agents for motility disorder of gastrointestinal tract, enhancement of relaxation of gastric fundus after meal in normal healthy subjects has been reported (Aliment Pharmacol. Ther. 1998: 12: 761-766), but clinical effect thereof on improving impaired gastric accommodation has not been reported.

### Summary of the Invention

The present inventors have extensively studied on improvement effect of various compounds on impaired gastric accommodation, and found that a compound described below has an excellent action to relax gastric fundus, or an action to alleviate impaired gastric accommodation, and has significant improving effect on feeling of early satiety and bloating. Furthermore, the present inventors have found that said compound has high safety, and thus completed the present invention.

Namely, the present invention provides a therapeutic agent for impaired gastric accommodation, wherein an active ingredient is a compound represented by the general formula (1): 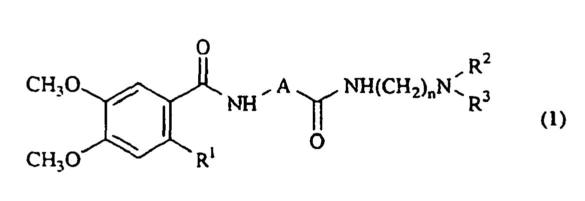 (wherein R¹ represents a hydrogen atom, a hydroxyl group or a halogen atom; A represents a furyl group, a thienyl group, a thiazolyl group or an oxazolyl group; R² and R³ each represents an alkyl group with 1 to 5 carbon atoms; and n represents an integer of 2 to 4), or an acid addition salt thereof.

Also, the present invention provides use of the above-described compound represented by the general formula (1) or an acid addition salt thereof to manufacture a therapeutic agent for impaired gastric accommodation.

Furthermore, the present invention relates to a treatment method for impaired gastric accommodation, characterized by administration of an effective dosage of a compound represented by the general formula (1), or an acid addition salt thereof.

### Detailed Description of the Invention

In the general formula (1), a halogen atom includes a fluorine atom, a bromine atom, a chlorine atom and an iodine atom, of which a chlorine atom is particularly preferable; A includes preferably a furyl group and a thiazolyl group, of which a thiazolyl group is particularly preferable; an alkyl group represented by R² and R³ includes a methyl group, an ethyl group, a n-propyl group, an isopropyl group, a n-butyl group and an isobutyl group, of which an isopropyl group is particularly preferable; and carbon number n is preferably 2, in particular.

Among these compounds represented by the general formula (1), preferred are 2-[N-(4,5-dimethoxy-2-hydroxybenzoyl)amino]-4-[(2-diisopropylaminoethyl)aminocarbonyl]-1,3-thiazole (compound a), 2-[N-(2-chloro-4,5-dimethoxybenzoyl)-amino]-4-[(2-diisopropylaminoethyl)aminocarbonyl]-1,3-thiazole (compound b), 2-[N-(4,5-dimethoxybenzoyl)amino]-4-[(2-diisopropylaminoethyl)aminocarbonyl]-1,3-thiazole (compound c), 2-[N-(4,5-dimethoxy-2-hydroxybenzoyl)amino]-4-[(2-diisopropyl-aminoethyl)aminocarbonyl]furan (compound d), or an acid addition salt thereof. Of them further preferable ones are 2-[N-(4,5-dimethoxy-2-hydroxybenzoyl)-amino]-4-[(2-diisopropylaminoethyl)aminocarbonyl]-1,3-thiazole (compound a), 2-[N-(2-chloro-4,5-dimethoxybenzoyl)amino]-9-[(2-diisopropylamino-ethyl)aminocarbonyl]-1,3-thiazole (compound b), or an acid addition salt thereof, with 2-[N-(4,5-dimethoxy-2-hydroxybenzoyl)-amino]-4-[(2-diisopropylaminoethyl)aminocarbonyl]-1,3-thiazole (compound a), or an acid addition salt thereof being more preferred.

These compounds represented by the general formula (1) (hereafter referred to as a compound (1)), or an acid addition salt thereof are described in WO96/36619 and JP-A-10-212271. The acid addition salt here includes an inorganic salt such as a hydrochloride, a hydrosulfate and a nitrate; and an organic acid salt such as a maleate, an acetate, a tartrate and a citrate, of which a maleate, a hydrochloride and a hydrate thereof are particularly preferable.

WO96/36619 and JP-A-10-212271 disclose that a compound (1) has activity to contract gastric antrum . On the contrary, the present inventors have found, by "Barostat test in dog", that a compound (1) has an excellent alleviating activity on gastric accommodatiion, that is not stomach contraction but relaxation of gastric fundus. By virtue of such activity, symptoms, including early satiety and bloating, can be alleviated significantly by administration of a compound (1) or an acid addition salt thereof.

High safety of a compound (1) or an acid addition salt thereof has been confirmed because no abnormality in ICR mouse after oral administration of 500 mg/kg was observed. The above described Cisapride is known to have a serious side effect such as extension of Q-T interval of stroke, but a compound (1) or an acid addition salt thereof has been confirmed not to have such adverse effect.

A compound (1) or an acid addition salt thereof can be formulated with a pharmaceutically acceptable carrier to prepare a composition for oral or parenteral administration. As for the composition for oral administration, a compound (1) or an acid addition salt thereof can be formulated with appropriate additives, for example, an excipient such as lactose, mannitol, cornstarch and crystalline cellulose; a binding agent such as cellulose derivatives, Arabic gum and gelatin; a disintegrant such as a calcium salt of carboxymethyl cellulose; a smoothing agent such as talc and magnesium stearate, to make tablet, powder, granule or capsule. These solid preparations can also be formulated as enteric coated drugs using coating base such as hydroxypropylmethyl cellulose phthalate, hydroxypropylmethyl cellulose acetate succinate, cellulose stearate phthalate or a methacrylate copolymer. As for the composition for parenteral administration, a solution for injection can be prepared by combination with, for example, water, ethanol, glycerin or a commonly used detergent. Suppository can also be prepared using an appropriate base for suppository.

Dosage of a compound (1) or an acid addition salt thereof depends on age, body weight, symptom, effect of treatment, an dosing method and dosing period, however, amount of oral administration is generally 0.1 to 2000 mg/day, preferably within the range between 50 to 1000 mg/day, and further in 1 to 3 portions a day.

### Examples

The present invention is described below in more detail by referring to Examples.

### Example 1 (Barostat test in dog)

### (1) Test method

A gastric fistula (KN-365, D-14-C from Natsume Seisakusyo Co., Ltd.) was installed permanently in the center of stomach anterior wall of an adult male mongrel dog, and was used for air-bag insertion.

The fistula-installed dog was abstained from food for not shorter than 18 hours, followed by opening the gastric fistula and washing inside stomach by infusion of saline into stomach, before starting experiment. This procedure was repeated 2 to 3 times as appropriate. The air-bag (maximal volume: about 750 mL) fabricated using a commercially available polyethylene bag was inserted into stomach through gastric fistula and fixed using a silicone plug, followed by connection to barostat instrument and starting measurement under nonrestraint condition. Volume change of the air-bag was recorded on both a recorder and a computer.

After insertion of the air-bag into stomach, the bag was once infused with air up to 500 mL, followed by immediate and complete air evacuation, and starting recording by adjusting initial inner pressure of the air-bag to be 6 ± 2 mmHg. After stabilization for not shorter than 15 minutes, 50 mL of a liquid test meal (12.5 kcal, Besvion^{RT} from Fujisawa Pharmaceutical Co., Ltd.) dissolved in warm water (30 to 40 °C) was administered into stomach. Five minutes after administration of the test meal, a solvent and test agents were administrated intravenously. Mean air-bag volumes (mL) for each 5 minutes before administration of the test meal, along with before and after administration of the test agents were calculated using a computer analyzing system to compare differences in mean air-bag volumes (mL) before and after administration of the test agents.

### (2) Results

Administration of the test meal into stomach caused a relaxation of gastric fundus and thus increased air-bag volume, which subsequently decreased with time.

In comparison with mean air-bag volume after administration of the test meal, that is, 5 minutes before administration of the test agents, change in air-bag volume after administration of the test agents were shown in Table 1. Air-bag volume decreased by 39.2 mL within 5 minutes after administration of the solvent. On the other hand, the administration of a compound a, hydrochloride, enhanced stomach relaxation and increased air-bag volume by 50.8 mL. Said expansion effect of a compound a surpassed that of cisapride.

**Table 1**

| Agent | Volume (mg/kg. i.v.) | Volume change of Air·bag (mL) | N |
|---|---|---|---|
| Solvent | - | -39.2±14.6 | 6 |
| Compound a, hydrochloride | 3 | 50.8±16.7* | 5 |
| Cisapride | 0.3 | 3.6±25.0 | 4 |
| Mean value ± standard | | | |
| * P < 0.05 vs solvent error group (Dunnett Two-Tailed test) | | | |

### Example 2 (barostat test in dog)

According to the method described in Example 1, barostat tests were carried out using a compound b, maleate, a compound c, hydrochloride, and a compound d, maleate. As shown by the results in Table 2, all of compounds b, c and d had an excellent activity of alleviating impaired gastric accommodation .

**Table 2**

| Agent | Volume mg/kg(i.v.) | Volume increase of air-bag, ΔmL | N |
|---|---|---|---|
| Compound b, Maleate | 1 | 29.7 | 1 |
| Compound c, hydrochloride | 1 | 58.2 | 1 |
| Compound d, Maleate | 3 | 11.4 | 1 |

### Example 3 (barostat test in human)

### (1) Recording method

A patient with functional dyspepsia, based on Rome II standard, was fasted overnight (for not shorter than 12 hours). Oral administration of 300 mg of a compound a, hydrochloride, and placebo has been conducted by a responsive doctor of clinical trial. Thirty minutes later, a polyvinyl adhesive plastic bag with dual ducts (1100 mL, maximal diameter: 17 cm, from Meditronic-Synetics Medical Ltd., Enfield, UK) was folded up to a small piece and introduced into stomach through mouth or nose, and the ducts were fixed on chin by an adhesive tape. The location of the bug in fundus of stomach was confirmed by radioscopy.

The polyvinyl tubing was connected to a computer controlled volume-replacement pressure regulator. This regulator can simultaneously monitor pressure and volume with sampling rate of 8 times per second, and also can load volume ramps and pressurizing steps with various rates. Trial subjects were kept in recumbent position and predetermined volume of air (300 mL) was infused for 2 minutes to expand the bug in stomach, and again the air was evacuated completely. After stabilization for 10 minutes, the trial subjects were allowed to keep relaxed by slightly skewing knees on a bed.

### (2) Test designing

After 30 minutes of adaptation period, minimum invasive gastric dilatation pressure (MDP), that is, the minimum pressure to give not less than 30 mL of the bag inner volume, was measured by increasing inner pressure by 1 mmHg per minute. This pressure (MDP) equilibrates intraabdominal pressure. Then, pressure was increased from the MDP stepwisely with isobaric expansions by each 2 mmHg. At each pressure increase step, corresponding inner volume of stomach was recorded, and kept for 2 minutes. At the end of each expansion step, patients were asked to express feeling of stimulation at upper abdomen by scores from 0 to 6 with verbal explanation. The expansion procedure was terminated when inner volume of the bag reached to 1000 mL or the patient complained discomfort or soreness (score: 5 or 6).

Further, after 30 minutes of adaptation period, the pressure level was adjusted to the MDP + 2 mmHg, and kept for 90 minutes. Thirty minutes later, a mixed liquid diet (200 mL, 300 kcal, protein 13 %, carbohydrate 39 %, Nutridrink^{™}, Nutricia) was given by oral ingestion, followed by measurement of stomach tension over further 60 minutes.

### (3) Data analysis

Average inner volume of the balloon for each 2 minutes expansion period was obtained from the recorded value. Threshold values of perceptivity and discomfort were obtained by analyzing the corresponding perceptual score to each expansion step. Threshold values of perceptivity and discomfort are defined as initial pressure at the perceptual score become not less than 1 and initial pressure at the perceptual score become not less than 5, respectively.

To evaluate stomach tension before and after meal, average volume of the balloon was measured continuously at every 5 minutes. The maximum relaxation value was obtained as difference between average volume before meal and the maximum volume after meal among average volumes measured at every 5 minutes after meal.

### Pain score

0 = no pain
1 = slight pain
2 = light pain
3 = medium pain
4 = high pain
5 = discomfort
6 = soreness

### (4) Results

Maximal relaxation value of stomach after meal, that is, food competence of stomach, is shown in Table 3.

**Table 3**

| | Placebo group N=15 | Compound a, hydrochloricle, administration group (300 mg, p.o.) N=17 |
|---|---|---|
| Max. relaxation volume before treatment (mL) | 345.1 | 295.5 |
| Max. relaxation volume after treatment (mL) | 296.1 | 313.4 |
| Change in max. relaxation volume (mL) | -55.3±106.3 | 24.3±172.3 |

As obvious from Table 3, a compound a, hydrochloride, significantly increased maximum relaxation value of stomach after meal.

### Industrial Applicability

Use of a therapeutic agent of the present invention improves relaxation of gastric fundus and impaired gastric accommodation and thus clearly alleviates symptoms caused by said disorders, including early satiety and bloating.

## Claims

1. Use of a compound represented by the general formula (1): 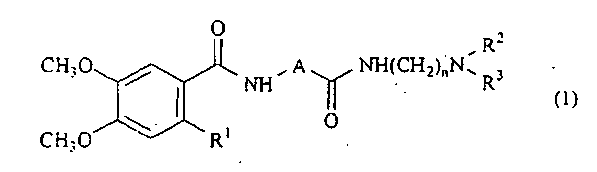 (wherein R¹ represents a hydrogen atom, a hydroxyl group, or a halogen atom; A represents a furyl group, a thienyl group, a thiazolyl group or an oxazolyl group; R² and R³ each represents an alkyl group with 1 to 5 carbon atoms; and n represents an integer of 2 to 4), or an acid addition salt thereof to manufacture a therapeutic agent for impaired gastric accommodation.

2. The use of claim 1, wherein A is a furyl group or a thiazolyl group.

3. The use of claim 1 or 2, wherein R² and R³ each represents an isopropyl groups and n is 2.

4. The use of any one of claims 1 to 3, wherein a compound represented by the general formula (1) is 2-[N-(4,5-dimethoxy-2-hydroxybenzoyl)amino]-4-[(2-diisopropylaminoethyl)aminocarbonyl]-1,3-thiazole or an acid addition salt thereof.

5. The use of any one of claims 1 to 4, wherein the gastric accommodation is improved by relaxation of gastric fundus.

## Patentansprüche

1. Verwendung einer Verbindung der allgemeinen Formel (1): 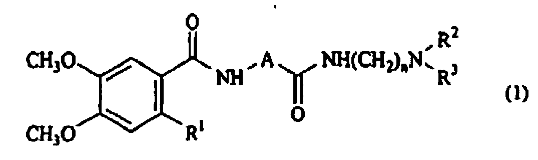 (worin R¹ für ein Wasserstoffatom, eine Hydroxylgruppe oder ein Halogenatom steht; A für eine Furylgruppe, eine Thienylgruppe, eine Thiazolylgruppe oder eine Oxazolylgruppe steht; R² und R³ jeweils für eine Alkylgruppe mit 1 bis 5 Kohlenstoffatomen steht; und n für eine ganze Zahl von 2 bis 4 steht), oder ein Säureadditionssalz derselben zur Herstellung eines therapeutischen Mittels für beeinträchtigte Magen-Akkomodation.

2. Die Verwendung gemäß Anspruch 1, worin A eine Furylgruppe oder eine Thiazolylgruppe ist.

3. Die Verwendung gemäß Anspruch 1 oder 2, worin R² und R³ jeweils für eine Isopropylgruppe steht und n 2 ist.

4. Die Verwendung gemäß einem der Ansprüche 1 bis 3, worin eine Verbindung der allgemeinen Formel (1) 2-[N-(4,5-Dimethoxy-2-hydroxybenzoyl)amino]-4-[(2-diisopropylaminoethyl)aminocarbonyl]-1,3-thiazol oder ein Säureadditionssalz derselben ist.

5. Die Verwendung gemäß einem der Ansprüche 1 bis 4, worin die Magen-Akkomodation durch Relaxation des Magenfundus verbessert wird.

## Revendications

1. Utilisation d'un composé représenté par la formule générale (1) : 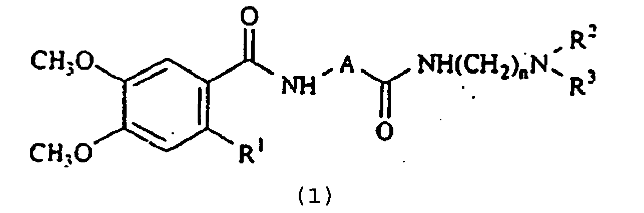 (dans laquelle R¹ représente un atome d'hydrogène, un groupe hydroxyle ou un atome d'halogène ; A représente un groupe furyle, un groupe thiényle, un groupe thiazolyle ou un groupe oxazolyle ; chacun de R² et R³ représente un groupe alkyle ayant de 1 à 5 atomes de carbone ; et n représente un entier de 2 à 4) ou d'un sel d'addition d'acide de celui-ci, pour fabriquer un agent thérapeutique destiné à une accommodation gastrique altérée.

2. Utilisation selon la revendication 1, dans laquelle A est un groupe furyle ou un groupe thiazolyle.

3. Utilisation selon la revendication 1 ou 2, dans laquelle chacun de R² et R³ représente un groupe isopropyle et n vaut 2.

4. Utilisation selon l'une quelconque des revendications 1 à 3, dans lequel le composé représenté par la formule générale (1) est le 2-[N-(4,5-diméthoxy-2-hydroxybenzoyl)amino]-4-[(2-diisopropylaminoéthyl)amino-carbonyl]-1,3-thiazole ou un sel d'addition d'acide de celui-ci.

5. Utilisation selon l'une quelconque des revendications 1 à 4, dans laquelle l'accommodation gastrique est améliorée par un relâchement du fundus gastrique.
